# EUROPEAN PATENT APPLICATION

(11) **EP 4 331 566 A1**
(43) Date of publication of application: **06.03.2024**
(21) Application number: 22744368.6
(22) Date of filing: 21.03.2022
(51) Int. Cl.: A61K 8/86, A61K 8/02, A61Q 11/00

(54) **MOUTH RINSE COMPOSITION INCLUDING POLYETHYLENE GLYCOL DERIVATIVE AS ACTIVE INGREDIENT FOR ALLEVIATING XEROSTOMIA**

(30) Priority: 22.03.2021 US 202117208075
(71) Applicant: Sunbio Inc., Gunpo-si, Gyeonggi-do 15849 (KR)
(72) Inventor: NHO, Kwang, Anyangsi Gyeonggi-do 14055 (KR); AHN, Minjung, Yongin-si Gyeonggi-do 16989 (KR); SOHN, Byunghee, Seoul 08325 (KR); HWANG, Seongu, Goyang-si Gyeonggi-do 10374 (KR); KIM, Donghwa, Gunpo-si Gyeonggi-do 15874 (KR)
(74) Representative: Plasseraud IP
(86) International application number: PCT/KR2022/003921
(87) International publication number: WO 2022/203316

(57) **Abstract**

The present invention relates to an oral rinse composition for alleviating xerostomia comprising a polyethylene glycol derivative as an active ingredient.

In addition, the oral rinse composition for alleviating xerostomia according to the present invention includes a polyethylene glycol derivative as an active ingredient so that it not only allows covalent bonding of the composition to the oral mucosa without irritation to increase the oral moisturizing capacity, but also allows easy manufacture and packaging by having a granule formulation, as well as being advantageous for storage and use.

## Description

### BACKGROUND

### 1. Field of the Invention

The present invention relates to an oral rinse composition for alleviating xerostomia comprising a polyethylene glycol derivative as an active ingredient.

### 2. Discussion of Related Art

Xerostomia is a disease in which the oral mucosa becomes dry due to a decrease in saliva secretion due to various causes. Saliva plays an important role in maintaining the main function of the oral cavity smoothly and protecting the oral mucosa, and therefore, when the amount of saliva secretion is reduced to cause xerostomia, extremely serious disorders occur, mainly drawing complaints about abnormalities in chewing function and in speech function, and causing the generation of severe bad breath and caries, and depending on the severity, depending on the degree, severe pain is experienced, for example, the burning feeling of the oral mucosa or the ulceration of oral mucosa.

The causes of xerostomia are congenital malformations such as salivary gland agenesis, autoimmune diseases such as Sjogren's syndrome or chronic graft-versus-host disease, and psychiatric disorders, diabetes, complications of radiation therapy, hepatitis C virus, chronic renal failure, AIDS, primary biliary cirrhosis, taking other medications, stress and mental illness, severe fever or diarrhea, dehydration due to large amounts of diuresis, when the salivary glands tube was blocked by calculus or cancer, body abnormalities such as infectious lesions of salivary glands such as epidemic parotitis have become the cause of temporary or long-term xerostomia. In addition, as the cause of human aging and menopausal disorders, saliva secretion is increased by various stimuli during waking activity, whereas the amount of saliva secretion is significantly decreased during sleep, and the proportion of patients who complained discomfort due to hypotasis occurring in the sleep reached 23%, and most of them were geriatric. (Thie et al., 2002).

For the treatment of xerostomia, among parasympathomimetic drugs, cholinergic drugs (pilocarpine (Salagen, MGI Pharmaceuticals., Minneapolis), cevimeline (Evoxac); 3 to 4 times postprandial doses per day, the effect thereof lasts 2-4 hours) are used to promote the secretion of saliva but also affect other parts of the body, thereby increasing the secretion of tears or nasal discharge or increasing sweat secretion. It is contraindicated in patients with asthma, acute iritis, narrow angle glaucoma, and glaucoma, and may have side effects in the case of cardiovascular disease, chronic bronchitis, and chronic obstructive disease. In addition, sugar-free gums and candy containing xylitol, lactoperoxidase, or glucose oxidase that promote saliva secretion are on sale. Chewing gum containing a mucosal ingredient continued to promote saliva secretion for a long time (Aagaard et al., 1992), and spraying the mucosal ingredient had a great effect on geriatric xerostomia (Momm and Guttenberger, 2002).

In the related arts, there is disclosed an oral rinse composition that can prevent and improve oral diseases by including eugenol and bamboo salt (Korea Patent Publication No. 10-2020-0050801), and there is disclosed a composition that can relieve bad breath by including a seaweed extract (Korean Patent No. 10-2313830).

However, since the related arts suggest that the main purpose is to kill harmful bacteria in the oral cavity and eliminate bad breath, there is a problem that it is difficult to expect an improvement effect as the duration of the relief of xerostomia is low because oral irritation is partially accompanied or a temporary hydration effect is exhibited.

In addition, a previous study by the present inventors (Korea Patent Registration No 10-1526258) discloses the inclusion of active ingredients of polyethylene glycol derivatives modified with various reactive groups as compositions for preventing, treating or ameliorating xerostomia that can bind to oral mucosa. Based on this, for actual commercialization, an oral rinse composition for alleviating xerostomia was completed based on the efficacy and safety results of each derivative and by confirming whether it can be applied to the human body.

### [Related art literature]

### [Patent Literature]

Korea Patent Publication No. 10-2020-0050801
Korea Patent Registration No. 10-2313830
Korea Patent Registration No. 10-1526258

### SUMMARY OF THE INVENTION

Under the above background, the present inventors have completed an oral rinse composition for alleviating xerostomia as a granule oral moisturizer which not only includes a polyethylene glycol derivative as an active ingredient and which can increase oral moisturizing maintenance by covalent bonding of oral mucosa without irritation, but also is easy to portable and easy to use.

Accordingly, an object of the present invention is to provide an oral rinse composition for alleviating xerostomia comprising a polyethylene glycol derivative as an active ingredient.

As a means for solving the above problems, the present invention provides an oral rinse composition for alleviating xerostomia comprising a polyethylene glycol derivative as an active ingredient.

Hereinafter, the configuration of the present invention will be described in detail.

The present invention relates to an oral rinse composition for alleviating xerostomia comprising a polyethylene glycol derivative as an active ingredient.

Polyethylene glycol is well known for its moisturizing effect. However, since it is applied in the oral mucosa and is easily washed off with water or saliva, the durability of the effect of alleviating xerostomia is low.

Therefore, in the present invention, as shown in Chemical Formula 1 below, this problem has been solved by modifying the terminal reactive group of polyethylene glycol to allow covalent bonding with oral mucosa epithelial cells, thereby allowing the polyethylene glycol derivative to adhere to oral mucosa epithelial cells for a long time.

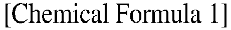

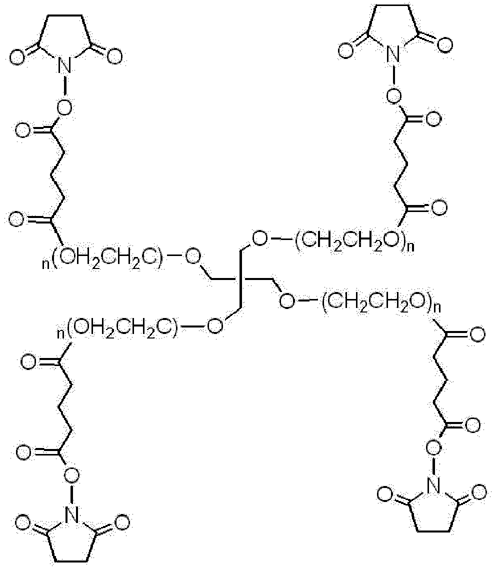

Through the previous studies of the present applicants, among various PEG derivatives, various PEG derivatives having a reactive group capable of covalent bonding with an amine group on oral mucosa epithelial cells have been developed. In the process of developing and researching products suitable for oral application using various PEG derivatives, the present inventors found that among them, halogen, amine (NH2), epoxide and maleimide reactive groups require long time for the amide coupling reaction with amine group of the cell, and thus, the oral application time should be prolonged. In addition, it was also revealed that the use of toxicants and by-products may remain in the reactive group synthesis process of the PEG derivative, and the odor property of the final finished PEG derivative causes the remaining fragrance caused by chemical synthesis and is unsuitable for oral application. In addition, it was confirmed that the aldehyde (CHO) reactive group requires a reducing agent (sodium cyanoborohydride) for the amide coupling reaction, and the nitrophenyl carbonate (NPC) reactive group produces harmful by-products (nitrophenol) in the amide reaction process, so it was not suitable for oral application.

Accordingly, the present inventors have completed the present invention by confirming that the use of a branched modified polyethylene glycol derivative having a -NHS (N-hydroxysuccinimide) reactive group of Chemical Formula 1 results in a fast amide reaction rate in oral application, no reagents required before and after the reaction, and no by-products, and there is no problem with discomfort and safety when applied in the oral cavity.

The present invention provides an oral rinse composition for alleviating xerostomia, comprising a polyethylene glycol derivative of the following Chemical Formula (1) as an active ingredient, an acidity adjusting agent and a flavoring agent; the polyethylene glycol derivative has a molecular weight of 8,000 to 15,000 Da, and the composition is the granule formulation.

As can be seen in the Examples below, it was confirmed that the polyethylene glycol derivative of Chemical Formula 1 had higher moisture absorption compared to the linear PEG derivative, which can enhance moisturizing maintenance because the polyethylene glycol derivative can trap more water molecules when binding to the oral mucosa.

In addition, the oral rinse composition for alleviating xerostomia according to the present invention is prepared in the granule formulation to improve storage stability such as moisture absorption and static electricity.

In one embodiment of the present invention, the granules may have a particle size of 200 to 1500 µm. For example, the granules may have a particle size of 300 to 1000 µm. The particle size of the granules is preferably 200 to 1500 µm for manufacturing (production) and packet packaging for one-time use and for stability during manufacturing and storage.

The composition of the granule formulation of the present invention is reconstituted in the form of a solution by dissolving it in water when used.

When 1 g of the composition of the granule formulation according to the present invention is quickly dissolved in 20 ml of water and applied orally, the dry oral mucosa is characterized by the formation of a water layer in which the polyethylene glycol derivative of formula (1) is tightly bound to the oral mucosa, thereby maintaining moisture retention for a long time compared to the temporary water layer of existing products, thereby maintaining moisture retention for a long time compared to the temporary water layer of existing products. Thus, the composition of the granule formulation of the present invention is a one-time use product that is mixed in an amount of 20 ml of water, then reconstituted into a solution, left in the mouth for about 30-60 seconds, and spit out, when the mouth feels dry, in particular, for xerostomia patients, an elderly person, and a common person before bedtime. Effects include oral moisturizing, caries prevention, and cleaning effects.

In the composition of the present invention, the acidity adjusting agent is included in an amount of 1 to 10 parts by weight, for example, 2 to 8 parts by weight, 3 to 7 parts by weight based on 100 parts by weight of the composition. Preferably, the acidity adjusting agent is included in an amount of 4 to 6 parts by weight.

Meanwhile, when the composition is dissolved in water for use and reconstituted in the form of a solution, the viscosity of the solution may be 0.001 Pa·s to 0.01 Pa·s. The viscosity of the solution can affect the convenience of users, application in the oral cavity, feeling of use, and the like, and therefore, the above range is suitable. The viscosity can not only improve palatability for oral application, but also prevent cytotoxicity and irritation occurring when the granules are directly administered. Alternatively, when the composition is dissolved in water in use and reconstituted in the form of a solution, the concentration of the solution may be 4 to 5%.

As the acidity adjusting agent is included, the composition is preferably dissolved in water for use to exhibit a pH of 7 to 8 when reconstituted in a solution form. According to the research results of the present inventors, a pH of 7 to 8 is a favorable condition for covalent bonding between the PEG derivative according to the present invention and an amine in the oral cavity. Furthermore, since the salivary pH of xerostomia patients is as low as 6.4 or less, and the xerostomia symptoms and salivary pH show a significant negative correlation, the pH of the composition for alleviating xerostomia symptoms is preferably pH 7 to 8.

As the flavoring agent, for example, a powdered food flavor suitable for oral application such as citrus, fruit, berry, vanilla, mint, etc. may be used, and the type is not particularly limited. The flavoring agent may be used in an amount of 1 to 10 parts by weight, for example, 4 to 10 parts by weight, based on 100 parts by weight of the composition, but is not limited thereto.

Each of the above ingredients included in the oral rinse composition according to the present invention may be included in the oral rinse composition of the present invention within a range that does not exceed the maximum amount prescribed in each country's food safety standards.

The present invention also provides a method of alleviating xerostomia, including applying the oral rinse composition for alleviating xerostomia to a subject in need thereof.

The application includes administering and rinsing an oral rinse composition for alleviating xerostomia reconstituted in solution form.

For example, when 1 g of the oral rinse composition of the granule formulation according to the present invention is quickly dissolved in 20 ml of water and applied orally, the polyethylene glycol derivative of Chemical Formula 1 is tightly bound to the oral mucosa to maintain moisture retention for a long time compared to the temporary water layer of existing products. Therefore, it can be usefully used for subjects or patients with xerostomia. The application may be carried out 1 to 3 times a day, but is not limited thereto. It can be applied at any time when the mouth feels dry, and it is particularly advantageous to apply it before bedtime.

In addition, the present invention provides a method of preparing an oral rinse composition for alleviating xerostomia.

Hereinafter, the configuration of the manufacturing method of the present invention will be described in detail.

The preparation method of the present invention may include preparing a polyethylene glycol derivative powder of Chemical Formula 1 having a molecular weight of 8,000 to 15,000 Da; mixing an acidity adjusting agent and a flavoring agent with the polyethylene glycol derivative powder, then transferring it to an airtight container and liquefying it at a high temperature; when the liquefaction is completed, transferring the airtight container to a low temperature to solidify; and when the solidification is completed, pulverizing to prepare granules.

In the preparation of the polyethylene glycol derivative of Chemical Formula 1 according to the present invention, -NHS was used as a reactive group, and it is suitable for mass production because NHS ester half-life is relatively long, the number of production processes is small and simple, and there is low risk in the production process.

In the step of preparing the polyethylene glycol derivative powder of Chemical Formula 1, the polyethylene glycol derivative preferably has a molecular weight of 8,000 to 15,000 Da. When the molecular weight is less than 8,000 Da or the molecular weight is more than 15,000 Da, there is a problem that not only moisture absorption is lowered, but also the crystallization and powdering processes are complicated during the production process, and the production yield is lowered.

In the step of selecting the polyethylene glycol derivative, the branched structure of the polyethylene glycol derivative is more advantageous than the linear structure. In the case of the branched structure, moisture absorption was higher, and since the number of derivative reactive groups that bind to the oral mucosa is large, it can retain more moisture for a longer period of time when combined with the oral mucosa to keep the oral cavity moist.

In the manufacturing method of the present invention, it may include adding an acidity adjusting agent according to pH 7 to 8.

The liquefying step may be carried out at 50 °C to 70 °C for 1 hour. At this time, when it is less than 50 °C, liquefaction is not sufficiently achieved, and when it exceeds 70 °C, the derivative molecules are partially decomposed and the effect is reduced.

The solidifying step may include primary hardening at room temperature for 1 hour; and secondary hardening at -20 °C (freezing).

When the particle size of the granules in the step of preparing the granules is 200 to 1500 µm, it is preferable for storage stability and to reduce static electricity during product packaging.

The present invention also provides a product for relieving xerostomia, including a container capable of measuring 15 ml to 30 ml of water per use and a packet containing a composition for alleviating xerostomia in a granule dosage form of 1 g to 1.5 g per use.

The product may include instructions for using a composition for alleviating xerostomia in a granule formulation packaged for single use including pouring and mixing the composition into a container measured an amount of water for single use to dissolve the composition, and then applying the composition to a dry mouth and spitting out the composition after gargling.

### [Effects of the Invention]

As the oral rinse composition for alleviating xerostomia according to the present invention includes a polyethylene glycol derivative of Chemical Formula 1 as an active ingredient, it not only allows covalent bonding of the composition to the oral mucosa without irritation to increase oral moisturization duration, but also allows easy manufacture and packaging by having a granule formulation, as well as being advantageous for storage and use.

### BRIEF DESCRIPTION OF THE DRAWINGS

FIGS. 1A and 1B show the results of nuclear magnetic resonance (NMR) analysis of the powder formulation and the granule formulation of the oral rinse composition for alleviating xerostomia according to the present invention.
FIG. 2 is a comparison result of surface static electricity according to the formulation of the oral rinse composition for alleviating xerostomia according to the present invention.

### DETAILED DESCRIPTION OF EXEMPLARY EMBODIMENTS

Hereinafter, the present invention will be described in detail by way of Examples. The following Examples merely illustrate the present invention and do not limit the scope of the present invention.

### Experimental Example 1. Moisture absorption Confirmation Test of PEG Derivatives for Oral Moisture Absorption

In order to identify and determine the PEG-OH structure having high oral moisture absorption, the moisture absorption rates of PEG derivatives having different structures were tested.

**Test materials:** As a PEG derivative for the moisture absorption test, a dry powder having a molecular weight of 3,000 Da or more rather than liquid (wax) PEG was used, and the test was carried out using powders of a linear structure of PEG (Chemical Formula A) having molecular weights of 3,400 Da and 10,000 Da, and a branched structure of 4arm PEG (Chemical Formula B) and 6arm PEG (Chemical Formula C) having molecular weights of 10,000 Da as described below.

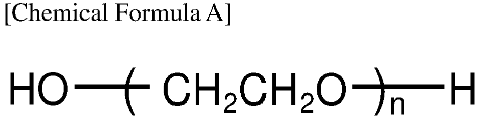

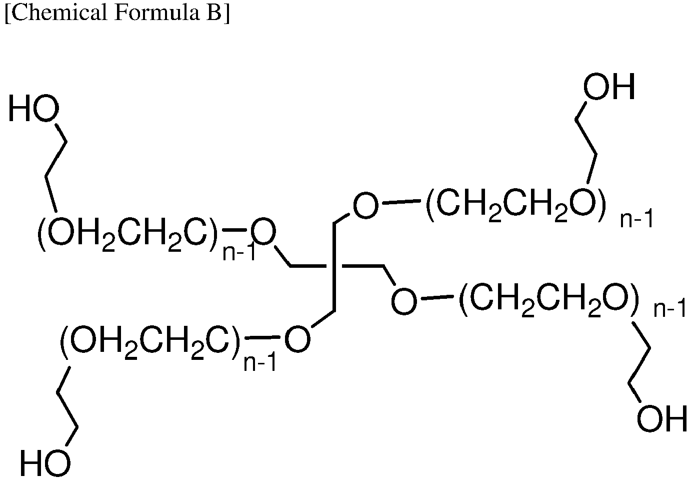

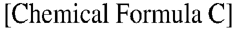

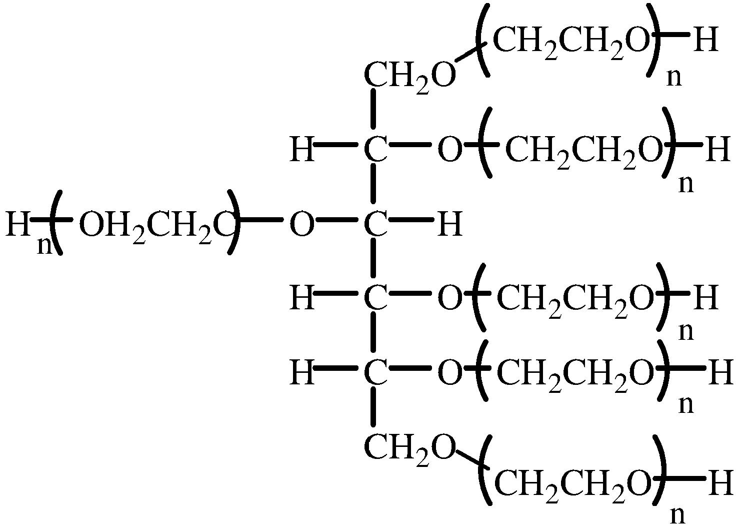

The test materials were purchased from Daechang Chemical, and for the test, the powder particles were uniformly pretreated by extraction and powdering processes using an organic solvent, and then the test was carried out.

Method for Measuring Moisture Absorption: The moisture absorption was confirmed by the weight of water vapor absorbed per mass of polyethylene glycol (PEG). After pretreatment by drying PEG in a desiccator at a temperature of 25 ± 1 °C, it was left for 24 hours under a condition of 60 ± 2% relative humidity at a temperature of 25 ± 1 °C, which is a moisture absorption condition, and the weight increase (mg) per 1g of PEG was defined as the moisture absorption of the PEG. The moisture absorption of each PEG was determined by comparing the weight difference before and after leaving the moisture absorption condition to be calculated as a percentage.

The results are shown in Table 2 below.

**[Table 1]**

| | Molecular Structure | Molecular Weight (Da) | Moisture absorption (%) | |
|---|---|---|---|---|
| | | | Numerical Value(%) | Mean(%) |
| Comparative Example 1 | Di-PEG-OH; P2OH | 3,400 | 10.00 | 10.14 |
| | | | 10.74 | |
| | | | 9.66 | |
| Comparative Example 2 | Di-PEG-OH; P2OH | 10,000 | 13.65 | 12.31 |
| | | | 11.99 | |
| | | | 11.29 | |
| **Example 1** | **4-arm PEG-OH ; P4OH** | **10,000** | **16.46** | **15.48** |
| | | | **13.85** | |
| | | | **16.14** | |
| Comparative Example 3 | 6-arm PEG-OH ; P6OH | 10,000 | 14.09 | 12.59 |
| | | | 11.95 | |
| | | | 11.72 | |

As shown above, it was confirmed that the linear Di-PEG-OH having a molecular weight of 3,400 Da (Comparative Example 1) showed the lowest moisture absorption rate of 10.14%, and 10,000 Da (Comparative Example 2) showed a rate of 12.31%, and the branched 4-arm PEG-OH having a molecular weight of 10,000 Da (Example 1) showed the highest moisture absorption rate of 15.48%.

As a result of confirming the moisture absorption of PEG-OH of linear and branched structures, the higher the molecular weight of the linear PEG-OH of the solid powder, the higher the moisture absorption (that is, the moisture absorption of the molecular weight of 10,000 Da (Comparative Example 2) is higher than that of 3,400 Da (Comparative Example 1)), and the moisture absorption of the branched PEG was higher when comparing the moisture absorption between the linear and branched PEG structures based on the same molecular weight of 10,000 Da. In particular, compared to the 6-arm PEG-OH (Comparative Example 3), the 4-arm PEG-OH (Example 1) structure exhibited the highest moisture absorption, so the 4 arm-PEG-OH (Example 1) was selected as the main ingredient of the present invention.

In addition to the branched 4arm-PEG-OH, 6arm-PEG-OH and 8arm-PEG-OH can be used for oral moisturizing, but no superiority to 4arm-PEG-OH could be confirmed in the synthesis/manufacturing process and moisturizing effect for PEG derivatives.

### Experimental Example 2. Moisture Content Confirmation Test By 4arm-Peg-OH Molecular Weight for Oral Moisture Absorption

According to the results of Experimental Example 1, it was confirmed that the branched 4arm-PEG-OH structure had high moisture absorption, and in order to confirm the moisture absorption according to the molecular weight of the same structure, a moisture content confirmation test according to 4arm PEG-OH of 10,000 Da, 20,000 Da, and 30,000 Da molecular weights was carried out. The test method was carried out by exposing 4arm-PEG-OH test materials for each molecular weight to a temperature of 25±1 °C and a relative humidity of 60±2% under moisture absorption conditions for 4 hours, and then analyzing the moisture content contained in each test material using a moisture meter (MS-70; METTLER TOLEDO, 400 W straight halogen lamp heating system with SRA filter and SHS weighting technology). That is, moisture absorption was confirmed using the difference in moisture content before and after exposure to moisture absorption conditions.

As shown in the table below, the moisture content and moisture absorption were measured according to the 4arm-PEG-OH structure having the molecular weights of 10,000 Da, 20,000 Da, and 30,000 Da. 4arm-PEG-OH PEG derivatives with a molecular weight of 5,000 Da or less (for example, 2,000 Da and 5,000 Da) were excluded from this test material because powdering was not easy in the production/synthesis process. 4arm-PEG-OH of each molecular weight was purchased from Daechang Chemical, and for the test, the powder particles were uniformly pretreated by extraction and powdering processes using an organic solvent, and then the test was carried out. The results are shown in Table 2 below.

**[Table 2]**

| | Molecular Structure | Molecular Weight (Da) | Moisture Content (%) | | | |
|---|---|---|---|---|---|---|
| | | | Before exposure (B) | After exposure (A) | A-B | AVR |
| **Example 1** | **4-arm PEG-OH ; P4OH** | **10,000** | 0.707 | 1.638 | 0.931 | **0.750** |
| | | | 0.753 | 1.400 | 0.647 | |
| | | | 0.807 | 1.480 | 0.673 | |
| Comparative Example 4 | 4-arm PEG-OH ; P4OH | 20,000 | 0.783 | 1.398 | 0.615 | 0.558 |
| | | | 0.724 | 1.368 | 0.644 | |
| | | | 0.764 | 1.180 | 0.416 | |
| Comparative Example 5 | 4-arm PEG-OH ; P4OH | 30,000 | 0.708 | 0.857 | 0.149 | 0.136 |
| | | | 0.691 | 0.811 | 0.12 | |
| | | | 0.695 | 0.834 | 0.139 | |

As shown in the above results, the smaller the molecular weight, the greater the difference in moisture content before and after 4 hours of exposure at 60% relative humidity. That is, it was found that the higher the molecular weight of 4arm-PEG-OH, the lower the moisture absorption.

That is, 4arm-PEG-OH (Example 1) of 10,000 Da among molecular weights 10,000 Da, 20,000 Da, and 30,000 Da of 4arm-PEG-OH showed the greatest difference (A-B) in moisture content before and after exposure to 60% relative humidity and was found to absorb a lot of moisture.

As described above, a 4arm-PEG-OH molecular weight 2,000 Da or 5,000 Da was excluded due to the difficulty of the final powder manufacturing process for the PEG derivative and the resulting low production yield. In the overall reaction process for PEG derivatization, the crystallization and powder manufacturing process affect the production yield in the chemical reaction process and the crystallization and recrystallization process using an organic solvent after filtration/concentration. In addition, it is an essential consideration because the production yield is lowered in the washing/recrystallization process to remove impurities with an organic solvent and the powdering process of the final even particles after drying. According to the results of Experimental Example 2, of 4arm-PEG-OH molecular weights 20,000 Da and 30,000 Da were not suitable for oral moisturizing because the moisturizing effect according to the increase in molecular weight was low.

For this reason, (Example 1) a 4arm-PEG-OH structure having a molecular weight of 10,000 Da was used as a main raw material for the synthesis and preparation of the PEG derivative, which is the main ingredient for oral moisturizing of the present invention.

### Experimental Example 3. Selection of PEG Derivative Reactive Group

In order to select a reactive group of a PEG derivative that forms a covalent bond with an amine group present in the oral mucosa, the half-life according to the reactive group of the PEG derivative and the number of production processes (number of steps) required for the synthesis of the reactive group were comparatively analyzed. When the half-life is short, the covalent bond with the amine group of the oral mucosa cannot occur because hydrolysis occurs quickly during dissolution of the composition in water. Therefore, it is difficult to use a reactive group with a short half-life for preparing an oral rinse composition for alleviating xerostomia.

The half-life was measured based on the UV absorbance of the N-hydroxy succinimide (NHS) group hydrolyzed in a buffer condition of 25°C and pH 8. The results are shown in Table 3 below.

**[Table 3]**

| | Molecular Structure | Reactive Group | Half-life (minutes)* | Number of Production Reaction Processes (Steps) |
|---|---|---|---|---|
| Comparative Example 6 | PEG-CH2CH2CH2CH2-CO2-NHS | Succinimidyl Valerate (SVA) | 33.6 | 4 |
| Comparative Example 7 | PEG-O-CO2-NHS | Succinimidyl Carbonate (SC) | 20.4 | 1 |
| **Example 2** | **PEG-O2C-CH2CH2CH2-CO2-NHS** | **Succinimidyl Glutarate (SG)** | **17.6** | **2** |
| Comparative Example 8 | PEG-O2C-CH2CH2-CO2-NHS | Succinimidyl Succinate (SS) | 9.8 | 2 |
| Comparative Example 9 | PEG-O-CH2-CO2-NHS | Succinimidyl Carboxymethylated (SCM) | 0.75 | 3 |
| Comparative Example 10 | PEG-O-CH2 CH2-CO2- NHS | Succinimidyl Butanoate (SBA) | 23.3 | 4 |
| Comparative Example 11 | PEG-O-CH2 CH2-CO2-NHS | Succinimidyl Propionate (SPA) | 16.5 | 5 |

As shown in the above results, succinimidyl carbonate (SC) ester (Comparative Example 7) had a simple production process of 1 step and a relatively long half-life, but there was risks and environmental risks of using phosgene gas in the manufacturing process. On the other hand, in the case of the derivative (Example 2) modified with succinimidyl glutarate (SG) according to the present invention, the amide reaction rate in oral application is fast, there are no reagents required before and after the reaction, and no by-products, and there is no problem with discomfort and safety in oral application. In addition, compared with other reactive groups, the half-life of Example 2 is relatively long, the number of production processes is small and simple, and the risk in the production process is low. Accordingly, a derivative (Chemical Formula 1) modified with succinimidyl glutarate (SG) was prepared through Preparation Example 1 below and used in subsequent experiments.

### <Preparation Example 1. Preparation of Polyethylene Glycol Derivatives of Chemical Formula 1>

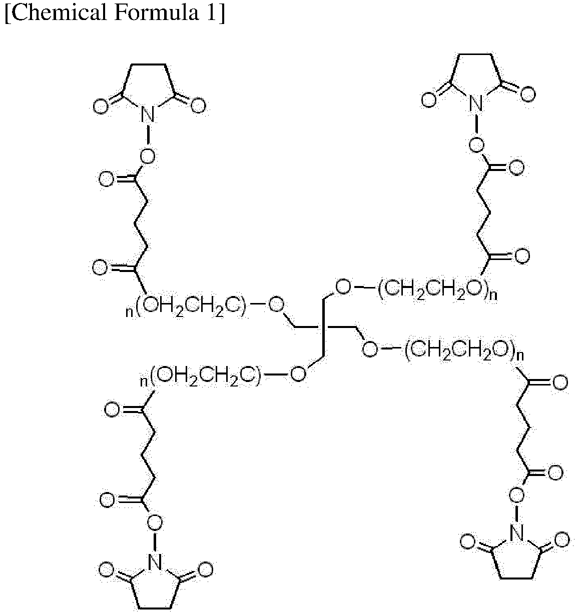

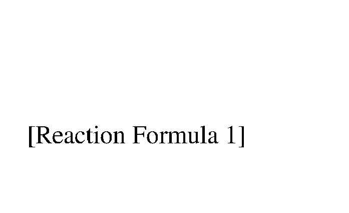

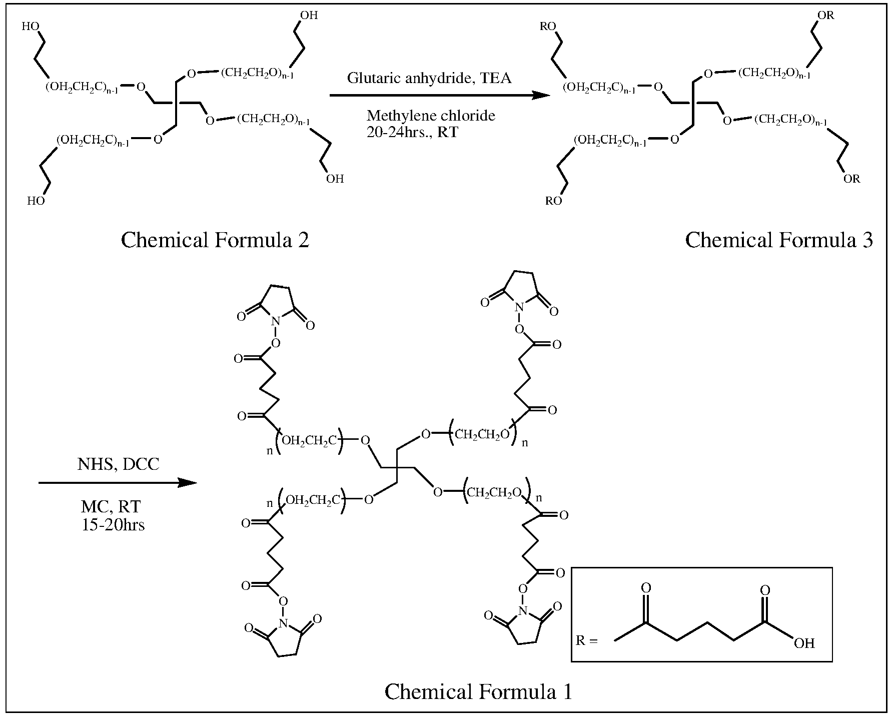

After dissolving the compound of Chemical Formula 2 in methylene chloride at room temperature, triethylamine was added. After adding glutaric anhydride to the reaction solution, stirring was performed at room temperature for 20 to 24 hours. The reaction solution was washed with a 14% ammonium chloride aqueous solution, and when the layers were separated, the lower organic solution layer was collected. The aqueous solution layer was extracted using methylene chloride. The combined organic solution layer was precipitated in diethyl ether after moisture was removed using magnesium sulfate and the solvent was concentrated. The precipitate was filtered and dried under vacuum at room temperature for 24 hours to obtain a compound of Chemical Formula 3.

The compound of Chemical Formula 3 was dissolved in methylene chloride, and N-hydroxy succinimide (NHS) and dicyclohexyl carbodiimide (DCC) were added thereto. The reaction solution was stirred at room temperature for 15 to 20 hours. After the reaction, dicyclohexyl urea (DCU) as a by-product was filtered using a glass filter, and the filtered solution was precipitated in diethyl ether after concentrating the solvent. After filtering the precipitate, it was dissolved in ethyl acetate at 55±5°C and recrystallized at 0-5°C for 15-17 hours. The recrystallized product was filtered, washed 3 times with diethyl ether, and dried under vacuum at room temperature for 24 hours to obtain a compound (n=57) of Chemical Formula 1 having a weight average molecular weight of 10,000.

### Experimental Example 4: Composition test using a pH adjusting agent

As a result of research by the present inventors, it was found that the conditions for forming a covalent bond between the amine group of the oral mucosa and the reactive group of the PEG-SG derivative of Chemical Formula 1 were pH 6.0 to 8.0.

However, when the PEG-SG derivative of Chemical Formula 1used as a main ingredient for oral moisturizing and the flavoring agent were dissolved in water, weak acidity (pH4.5), which was not suitable for the above pH conditions, was exhibited.

In order to adjust the composition of the present invention to a pH condition suitable for covalent bonding in the oral cavity, an acidity adjusting agent (sodium bicarbonate) used as a food additive was added to determine the amount of the acidity adjusting agent so that the pH was 6.0 to 8.0".

**Test Materials:** As shown in Table 4 below, the acidity adjusting agent (sodium bicarbonate) was prepared by adding 0 (control), 1, 5, and 10% of the product content (1.5 g). A pH test was carried out using three 1g samples for each composition.

**[Table 4]**

| | Polyethylene Glycol Derivative of Chemical Formula 1 | Flavoring Agent | Acidity Adjusting Agent | Total |
|---|---|---|---|---|
| Comparative Example 12 | 93.3% (1.4g) | 6.7% (0.1g) | 0% (0.000g) | 100% (1.5g) |
| Example 3 | 92.3% (1.385g) | 6.7% (0.1g) | 1% (0.015g) | 100% (1.5g) |
| Example 4 | 88.3% (1.325g) | 6.7% (0.1g) | 5% (0.075g) | 100% (1.5g) |
| Example 5 | 83.3% (1.25g) | 6.7% (0.1g) | 10% (0.150g) | 100% (1.5g) |

**Test Methods:** To accurately measure the pH of the prepared samples, pH meter calibration was carried out in 3 sections (pH 4.0, 7.0, 10.0 standard buffer), and the allowable parameter pH gradient was 90 to 105% and 0 ± 30 mV (0.5 at 25 °C pH units) were checked. After completely dissolving 1 g of each test material in 20 ml of distilled water, the pH was checked.

The results are shown in Table 5 below.

**[Table 5]**

| | **pH** | | | **Mean pH** |
|---|---|---|---|---|
| Comparative Example 12 | 4.5 | 4.4 | 4.5 | 4.5 |
| Example 3 | 7.0 | 7.1 | 7.0 | 7.0 |
| Example 4 | 7.4 | 7.5 | 7.5 | 7.5 |
| Example 5 | 7.8 | 7.8 | 7.9 | 7.8 |

As shown in the above results, the mean pH of each sample to which 1, 5, and 10% of the acidity adjusting agent was added was 7.0, 7.5, and 7.8. As a result, it was confirmed that 1 to 10% of the addition amount of the acidity adjusting agent was in an amount range suitable for covalent pH 6.0 to 8.0 conditions.

However, as a result of sensory evaluation, the composition in which the acidity adjusting agent was added in an amount of 10% or more (Example 5) was excluded because it caused a bitter taste and irritation to the tongue when applied orally. In subsequent experiments, a composition (Example 4) having an acidity adjusting agent addition amount of 5% was prepared and used according to Preparation Example 2 below.

### <Preparation Example 2. Preparation of Oral Rinse Composition for Alleviating Xerostomia>

An oral rinse composition for alleviating xerostomia, including the polyethylene glycol derivative of Chemical Formula 1, an acidity adjusting agent and a flavoring agent, was prepared in various formulations, and cytotoxicity (human safety), human applicability such as moisturizing effect (functionality), and ease of handling in a commercialization process, product stability, etc. were evaluated through the following Experimental Examples.

### <Preparation Example 2-1. Preparation of Oral Rinse Composition for Alleviating Xerostomia in Powder Formation>

Each of 89 parts by weight of a polyethylene glycol derivative, 5 parts by weight of sodium hydrogen carbonate as an acidity adjusting agent, and 6 parts by weight of mint as a flavoring agent was added to a V-type mixer, and mixed at 20 to 25rpm for 15 minutes to prepare a finished composition in powder form.

### <Preparation Example 2-2. Preparation of Oral Rinse Composition for Alleviating Xerostomia in Liquid Formation>

1.5g of the powder composition of Preparation Example 2-1 was dissolved in 30ml of water to prepare a liquid composition.

### <Preparation Example 2-3. Preparation of Oral Rinse Composition for Alleviating Xerostomia in Granular Formation>

An airtight container accommodating the powder composition prepared in Preparation Example 2-1 was liquefied at 60°C for 1 hour. When the liquefaction was completed, the airtight container was transferred to room temperature and primarily hardened for 1 hour, and then secondarily hardened at -20°C to solidify. When the solidification was completed, the solidified product was pulverized to a particle size of 355 to 1400µm.

### Experimental Example 5. Cytotoxicity Test

The cytotoxicity test is a test to confirm the safety of the composition according to the present invention, and the presence and degree of a cytotoxic reaction in cultured cells (L-929) was evaluated. A good laboratory practice (GLP) institution was commissioned to perform the test according to each test method presented in ISO 10993.

### Test Materials:

As shown in Table 6 below, each test solution was prepared using a powder formulation (A) without an acidity adjusting agent, a powder formulation (B, Preparation Example 2-1) containing an acidity adjusting agent, and a liquid formulation test material (C, Preparation Example 2-2) in which a powder formulation (B) was dissolved in 30 ml of water.

**[Table 6]**

| | Powder formulation (A) | Powder formulation (B) | Liquid formulation (C) |
|---|---|---|---|
| Classification | Comparative Example 13 (Same as Comparative Example 12) | Comparative Example 14 (Same as Example 4) | Example 6 |
| Polyethylene Glycol Derivative (4arm-PEG-SG) | 1.4 g | 1.325 g | 1.325 g |
| Flavoring Agent | 0.1 g | 0.1 g | 0.1 g |
| Acidity Adjusting Agent | - | 0.075 g | 0.075 g |
| Total | 1.5 g | 1.5 g | 1.5 g in 1Oz(30ml) DW |

### Cytotoxicity Test Method

**5-1. Test Method of Powder Sample:** In the extract (E-MEM +5% FBS), the powder test material and the control were dissolved at a concentration of 0.2 g/ml, and dissolved for 24 ± 2 hours to prepare a test solution. The culture medium of cell L-929 was removed from the plate well, replaced with 1 mL of test solution and control medium (extract solution), and then the cells were cultured. During culturing, microscopic examinations were carried out every 24, 48, 72 ± 4 hours to evaluate cytotoxicity. Positive and negative controls were also used in the test.

**5-2. Test Method of Liquid Sample:** A liquid test solution was prepared in which a dilution solution (2X E-MEM + 10% FBS) and the liquid test material were combined/diluted in a 1:1 ratio. The culture medium of cell L-929 was removed from the plate well, replaced with 1 mL of test solution and control medium (diluent), and then the cells were cultured. During culturing, microscopic examinations were carried out every 24, 48, 72 ± 4 hours to evaluate cytotoxicity. Cadmium chloride (100uM CdCl2) as a positive control and E-MEM + 5% FBS as a negative control were also used in the test.

As the evaluation criteria of the cytotoxicity test, a score of 0 to 4 was recorded by evaluating the morphological change of cells and the degree of change in viable cells according to lysis or separation. Scores 3 and 4 were judged to be cytotoxic, and scores of 1 and 2 were judged to be non-cytotoxic.

The results are shown in Tables 7 and 8 below.

**[Table 7]**

| Classification | Cytotoxicity Score | | | | | | | | |
|---|---|---|---|---|---|---|---|---|---|
| | Comparative Example 13 | | | Comparative Example 14 | | | Example 6 | | |
| | 24hr | 48hr | 72hr | 24hr | 48hr | 72hr | 24hr | 48hr | 72hr |
| Test Material | 4/4/4 | 4/4/4 | 4/4/4 | 4/4/4 | 4/4/4 | 4/4/4 | 0/0/0 | 0/0/0 | 0/0/0 |
| Positive Control | 4/4/4 | 4/4/4 | 4/4/4 | 4/4/4 | 4/4/4 | 4/4/4 | 0/0/0 | 0/0/0 | 0/0/0 |
| Negative Control | 0/0/0 | 0/0/0 | 0/0/0 | 0/0/0 | 0/0/0 | 0/0/0 | 0/0/0 | 0/0/0 | 0/0/0 |
| Cell Control | 0/0/0 | 0/0/0 | 0/0/0 | 0/0/0 | 0/0/0 | 0/0/0 | 0/0/0 | 0/0/0 | 0/0/0 |

As shown in the above results, as a result of confirming the cytotoxicity test for each composition and both formulations, for Comparative Examples 13 and 14, a cytotoxicity score of '4' was recorded and strong cytotoxicity was exhibited, and Example 6 did not exhibit any cytotoxicity with a cytotoxicity score of '0'. Example 6 includes an acidity adjusting agent, and by dissolving the composition in 30 ml of water and applying it to the cells as a liquid, it was confirmed as a safe composition and formulation that did not affect the morphological change and survival of L-929 cells.

**[Table 8]**

| Classification | Powder Formulation (a) | Powder Formulation (b) | Liquid Formulation (c) |
|---|---|---|---|
| | Comparative Example 13 | Comparative Example 14 | Example 6 |
| Amount of test material | 1.5 g | 1.5 g | 0.05 g/ml (1.5 g/30ml in DW) |
| Cytotoxic sample concentration | Test Material Extraction Type | | Test Material Dilution Type* |
| | 0.2g/ml in media | 0.2g/ml in media | 0.025g/ml in media |
| Cytotoxicity result | Cytotoxicity. | Cytotoxicity. | No Cytotoxicity. |

As shown in the above results, since the powder compositions (Comparative Examples 13 and 14) exhibit cytotoxicity, it is not suitable to directly apply the dry powder formulation to a xerostomia patient with a small amount of saliva secretion.

Therefore, after changing to a liquid formulation by adding water to the powder formulation of the present invention, no toxicity was observed as the concentration of the test material for the cytotoxicity test was reduced from 0.2 g/ml to 0.025 g/ml (1.5 g/30 ml x 1/2). In addition, unlike applying a powder composition to the dry oral mucosa, which gives irritation to the sensitive mucous membrane, when the powder was reconstituted into a liquid formulation, moisture that the oral cavity lacked was additionally supplied, and the expected effect of increasing the moisturizing effect was exhibited by allowing the patient to easily apply the product in the oral cavity.

### Experimental Example 6. Moisturizing Effect Confirmation Test According to Cell Viability

By applying the composition of the present invention to human epithelial cells, a moisturizing layer is formed by covalent bonding of the main ingredient PEG derivative to the cell surface, and an in vitro test was carried out to confirm cell viability by exposing the cells to a dry environment.

### Test Materials:

Example 6 of the present invention, in which cytotoxicity safety was confirmed in Experimental Example 5, and Hydris Oral Rinse, a product for relieving xerostomia manufactured by Colgate, were used as test materials. Both test materials are liquids in the same formulation. As a negative control, PBS (phosphate-buffered saline; Gibco) was used.

**[Table 9]**

| | **Hydris^{™} Oral Rinse** | **Example 6** |
|---|---|---|
| **Main Ingredient Moisturizer (Main Ingredient)** | Glycerin, Polyethylene Glycol | PEG Derivative (4arm-PEG-SG) |
| **Other** | Sorbitol, Sodium Saccharin, Sucralose/Cellulose Gum, Xanthan | None |
| **(Sweetener, Thickener, Surfactant, Preservative)** | Gum, Carbomer/Poloxamer 407/Cetylpyridinium Chloride, Sodium Benzoate | |
| **Buffer** | Disodium Phosphate, Sodium Phosphate | Sodium Bicarbonate |
| **Coloring Agent, Flavoring Agent** | FD&C Blue 1, Mint | Mint |

### Test Method:

Using human pharynx epithelial cells, the composition of the present invention, Hydris^{™} Oral Rinse of Colgate, and phosphate-buffered saline (PBS) were applied to the cells at 37 °C for 15 minutes and cultured, then the test material was removed and washed with PBS, the cells were exposed to dry conditions of 30% humidity at 25 °C, and cell viability was measured. The effect of the treated test materials to protect cells by reducing apoptosis due to drying was confirmed by cell viability. Cells were treated with PBS as a positive control, and the viability of cells not exposed to dry conditions was set to 1 (100%), and cell viability according to treatment with a test material was expressed as a percentage. Cell viability was analyzed using Cell Counting Kit 8 (CCK8; Dojindo Molecular Technologies, Inc., Rockville, MD, USA). This kit is used to quantify the number of living cells by generating orange formazan dye during bioreduction in the presence of electron carriers using a water-soluble tetrazolium salt.

The results are shown in Table 10 below.

**[Table 10]**

| Classification | **Non-Dry Condition (Positive Control)** | **Dry Condition** | | |
|---|---|---|---|---|
| | **PBS** | **PBS** | **Hydris^{™}** | **Example 6** |
| | (N = 8) | (N = 16) | (N = 8) | (N = 8) |
| Mean | 100 | 11.6 | 12.0 | **71.9** |
| Median | 101.9 | 4.6 | 11.5 | **81.5** |
| SD | 7.34 | 17.06 | 3.38 | **47.26** |

As shown above, the mean value of the cell viability of Example 6 of the present invention based on the cell viability (%) of the positive control was 71.9% (median: 81.5%), showing the highest cell viability, and the Hydris product of GSK showed a relatively low viability with a mean value of 12.0% (median: 11.5%).

Because the moisturizing ingredient remained in the cells even after cell washing by covalent bonding of the main ingredient PEG derivative of Example 6 and oral cells to maintain the effect of the moisturizing coating, cells are washed and then exposed to a dry environment to minimize cell death due to drying to maintain cell viability. In other words, the composition including the existing moisturizer is washed off by talking or ingestion of drinks/food after oral application, so it has a limited role as a moisturizer, but the moisturizing effect is temporary, and therefore, it must be frequently applied intraorally. However, the main ingredient of the PEG derivative of the present invention is for maintaining a moisturizing effect for a long time without being washed away by physical stimulation by covalent bonding with cells.

### Experimental Example 7. Reactive Group Activity and Electrostatic Test of the Main Ingredient 4arm-Peg-Sg Derivative

Common PEG derivatives are prepared in the form of final powder (powder type) through chemical reaction, synthesis, a manufacturing process, and a recrystallization process after crystallization and washing. These powdered PEG derivatives are mixed with additives, and the recovered mixture is filled into the hopper of the equipment for fixed quantity (1 g) packet packaging, and then the powder is supplied to the scale (load cell) from the vibrating plate and the weight is measured, and the fixed quantity is filled in aluminum packets. However, in the case of a powder formulation such as Preparation Example 2-1, a loss due to static electricity occurs during the weighing, mixing and recovery of the mixture at the beginning of the production process, and for the same reason, it was found that the recovery rate was lowered.

Therefore, changes in reactive group activity and whether static electricity was generated were analyzed by changing the physical form of the granule formulation of Preparation Example 2-3 to solve the static electricity problem in the manufacturing process caused by the physical form, that is, powder, of 4arm-PEG-SG, which is the main ingredient of the PEG derivative of the present invention.

**Test materials:** As in the previous experimental examples, 4arm-PEG-SG, a PEG derivative in powder form, and a sample in the form of granules were prepared.

### Test Method:

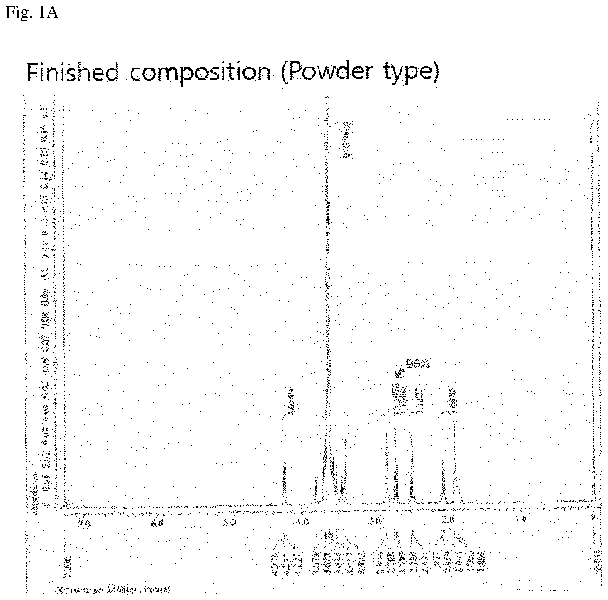

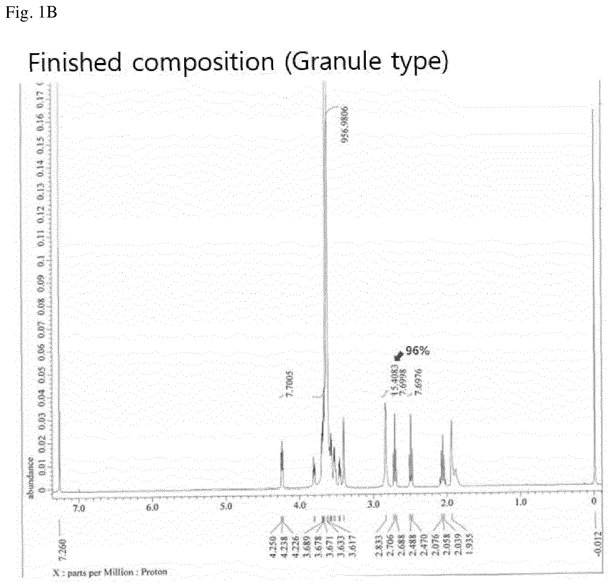

Prior to the electrostatic test, it was confirmed by nuclear magnetic resonance (NMR) analysis that there was no problem in the reactive group activity and stability of the PEG derivative even after the physical state change of melting and solidifying 4arm-PEG-SG in powder form. FIGS. 1A and 1B are the results of nuclear magnetic resonance (NMR) analysis of the powder formulation and the granule formulation of the oral rinse composition for alleviating xerostomia according to the present invention, confirming that there is no problem in the activity and stability of the reactive group regardless of the change in physical state.

The electrostatic test was measured using an electrostatic meter (EYE-02) capable of measuring the surface electrostatic force of the main ingredient PEG derivatives of the two formulations. 50 g and 100 g of each of the PEG derivatives, the main ingredient of the powders and granules, were placed in a PET storage pack (vinyl pack) used for weighing in the manufacturing process, and the surface electrostatic force was measured and compared.

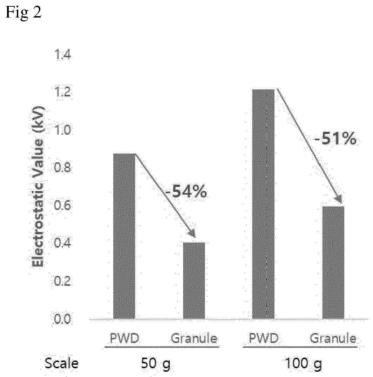

As shown in Figure 2, as a result of confirming and comparing the surface electrostatic force generated depending on the physical form, that is, powders and granules of the PEG derivative 4arm-PEG-SG, it was confirmed that the surface static electricity of the powder decreases by about 50% or more when the morphology changes into granules.

As a result, in the case of the granule formulation of Preparation Example 2-3, the loss in the hopper due to static electricity was reduced and packet filling was facilitated, unlike the powder formulation of Preparation Example 2-1.

### Experimental Example 8. Stability Confirmation Test According to the formulation of the Final Finished Composition

A test to confirm product stability according to the formulation was carried out as follows.

**Test Materials:** An oral rinse composition of the powder formulation of Preparation Example 2-1 (Comparative Example 15) and the granule formulation of Preparation Example 2-3 (Example 7) of the present invention was prepared, and 1 g of each was packaged in an aluminum packet.

**Stability Test Method:** 1g of the finished composition in powder and granule form was filled in an aluminum packet (9cm x 2cm), sealed with a heat adhesive, and stored in a thermo-hygrostat chamber with a relative humidity of 60% ± 5% at 25 ± 2°C. Stability test analysis was performed on days 0, 1, 2, 3, 4, 5, 6, 7, 8 and 10 of storing the sample, 10mg of the analyzed sample was weighed, put into an NMR tube and dissolved in 0.62mL of CDCl₃, and analyzed by 1H-NMR (400 MHz). The analysis method confirmed the reactive group activity of the main ingredient 4arm-PEG-SG derivative by the succinimidyl group peak of 2.80-2.90ppm (parts per million; proton) of the NMR spectrum. That is, since 4arm-PEG-SG is the main ingredient that binds to the oral mucosa and provides a moisturizing effect, the functional group (succinimidyl group) activity of 4arm-PEG-SG, which covalently bonds with the amine (NH₂) group of the oral mucosa, was analyzed by 1H-NMR, and the stability of the finished composition was confirmed. The stability of the finished composition was judged as "Stable" when the NMR activity value of the main ingredient 4arm-PEG-SG was 90% or more, and "Not stable" when it was less than 90%.

The results are shown in Table 11 below.

**[Table 11]**

| **Test Material** | **Result of NMR (%) Analysis According to the Test Period** | | | | | | | | | |
|---|---|---|---|---|---|---|---|---|---|---|
| | Day 0 | Day 1 | Day 2 | Day 3 | Day 4 | Day 5 | Day 6 | Day 7 | Day 8 | Day 10 |
| Comparat ive Example 15 **(Powder)** | 96.0 3 | 95.9 7 | 95.4 4 | 92.1 5 | 88.1 4 | N/A | N/A | N/A | N/A | N/A |
| | 96.0 7 | 95.9 5 | 95.0 0 | 92.1 8 | 87.0 1 | N/A | N/A | N/A | N/A | N/A |
| | 96.0 4 | 95.9 6 | 95.3 4 | 92.2 5 | 87.0 4 | N/A | N/A | N/A | N/A | N/A |
| | Stable | | | | Not stable | | | | | |
| **Example 7 (Granule )** | 96.5 1 | 96.3 6 | 96.2 1 | 95.5 0 | 93.9 6 | 93.6 3 | 90.2 6 | *90.2 1 | 89.2 5 | 86.88 |
| | 96.4 1 | 96.4 0 | 95.8 1 | 95.4 8 | 94.0 8 | 92.2 8 | 90.2 9 | 88.81 | 86.9 4 | 80.84 |
| | 96.3 3 | 96.3 2 | 95.9 7 | 94.5 5 | 93.8 5 | 92.6 6 | 92.6 8 | 88.22 | 87.9 5 | 83.94 |
| | Stable | | | | | | | Not stable | | |

| | | | | | | | | | | |
|---|---|---|---|---|---|---|---|---|---|---|
| *On day 7, one batch among the three batches satisfies the standard (90% or more), but the mean value of the three batches is below the standard, so it is judged as Not stable. | | | | | | | | | | |

As shown in the above results, as a result of confirming the stability test under severe conditions (25 ± 2°C, relative humidity 60% ± 5%) according to the formulation of the finished composition, the reactive group activity of the main ingredient 4arm-PEG-SG derivative showed stable results of 92.15%, 92.19%, and 92.25% on day 3 for composition in the form of a powder (Comparative Example 15), and 90.26%, 90.29%, and 92.68% on day 6 for composition in the form of a granule (Example 7).

When the formulation of the finished composition was in the form of powder (Comparative Example 15), the granule finished composition (Example 7) showed stable results for 6 days compared to the results stable for 3 days under severe conditions, and it was confirmed that the stability was improved. The formulation of the composition is changed to a granule form, and compared to a powder form, the exposure area to the air is reduced, and it appears that the absorption of moisture in the air according to the decrease in exposure is reduced and the stability is increased.

Therefore, the formulation of the final finished composition was determined to be granule. A product for alleviating xerostomia including a container capable of measuring the amount of water per use and a packet packaged with a composition for alleviating xerostomia in the form of granules per use was developed as a final product.

## Claims

1. An oral rinse composition for alleviating xerostomia,
the composition comprises a polyethylene glycol derivative of the following Chemical Formula (1) as an active ingredient, an acidity adjusting agent and a flavoring agent,
the polyethylene glycol derivative has a molecular weight of 8,000 to 15,000 Da,
the composition is in the form of a granule formulation:

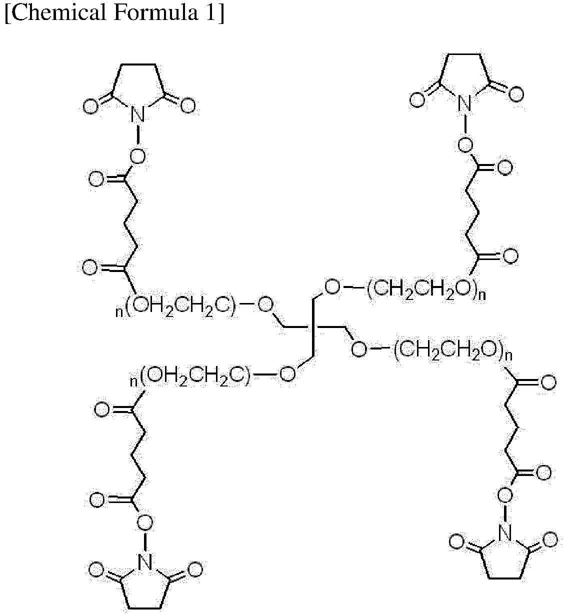

2. The oral rinse composition of claim 1, wherein the granules have a particle size of 200 to 1500µm.

3. The oral rinse composition of claim 1, wherein the composition is dissolved in water and reconstituted in the form of a solution for use.

4. The oral rinse composition of claim 1, wherein the acidity adjusting agent is comprised in an amount of 1 to 10 parts by weight based on 100 parts by weight of the composition.

5. The oral rinse composition of claim 3, wherein the composition exhibits a pH of 7 to 8 when dissolved in water and reconstituted in a solution form for use.

6. The oral rinse composition of claim 3, wherein the solution exhibits a viscosity of 0.001 to 0.01 Pa·s.

7. A method of alleviating xerostomia, comprising applying the oral rinse composition for alleviating xerostomia of claim 1 to a subject in need thereof.

8. The method of claim 7, wherein the application comprises administering and rinsing an oral rinse composition for alleviating xerostomia reconstituted in solution form.

9. The method of claim 7, wherein the application is carried out 1 to 3 times a day.

10. A method of preparing an oral rinse composition for alleviating xerostomia of claim 1, comprising:
preparing a polyethylene glycol derivative powder of Chemical Formula 1 having a molecular weight of 8,000 to 15,000 Da;
mixing an acidity adjusting agent and a flavoring agent with the polyethylene glycol derivative powder, then transferring it to an airtight container and liquefying it at a high temperature;
when the liquefaction is completed, transferring the airtight container to a low temperature to solidify; and
when the solidification is completed, pulverizing to prepare granules;

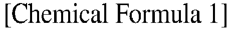

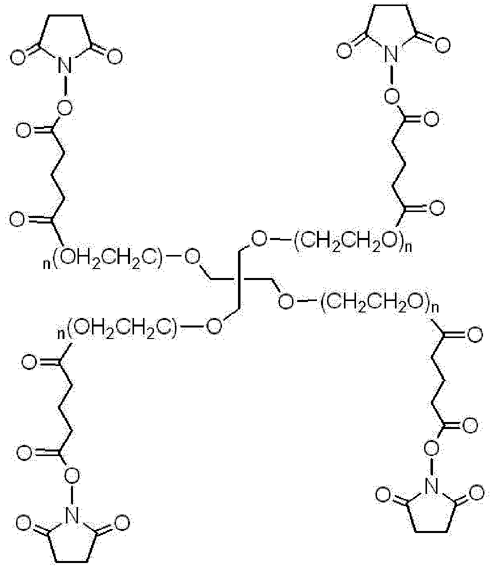

11. The method of claim 10, comprising adding an acidity adjusting agent to bring the pH to 7 to 8.

12. The method of claim 10, wherein the liquefying is carried out at 50 to 70 °C for 1 hour.

13. The method of claim 10, wherein the solidifying comprises:
primary hardening for 1 hour at room temperature; and
secondary hardening at -20 °C.

14. A product for relieving xerostomia, comprising a container capable of measuring 15 to 30 ml of water per use and a packet packaged with an oral rinse composition for alleviating xerostomia of 1 to 1.5 g of the granule formulation of claim 1 per use.

15. The product of claim 14, wherein the amount of water per use is 20 ml, and the oral rinse composition per use is 1 g.
